# FASCICULE DE BREVET EUROPEEN

(11) **EP 2 769 643 B1**
(45) Date de publication et mention de la délivrance du brevet: **11.11.2020**
(21) Numéro de dépôt: 14305239.7
(22) Date de dépôt: 20.02.2014
(51) Int. Cl.: A47C 15/00, A47C 3/16, A47C 5/12, A47C 9/00, A61F 5/01

(54) **Siège ergonomique notamment pour une personne souffrant de troubles du positionnement et/ou du comportement**
Ergonomischer Sitz vor allem für eine Person, die von Störungen der Positionierung und / oder des Verhaltens betroffen ist
Ergonomic seat especially for a person suffering from disorders of positioning and / or behavior

(30) Priorité: 22.02.2013 FR 1351575
(43) Date de publication de la demande: 27.08.2014
(73) Titulaire: Bulle Confort & Orthopedie Sarl, 68500 Guebwiller (FR)
(72) Inventeur: Rosello, Lionel, 68360 Soultz (FR)
(74) Mandataire: Koelbel, Caroline

(56) Documents cités:
- AU-A4- 2011 100 012
- CA-A1- 2 578 639
- US-A- 5 490 717

## Description

### Domaine technique :

La présente invention concerne un siège ergonomique notamment pour une personne souffrant de troubles du positionnement et/ou du comportement, ce siège étant agencé pour soutenir une personne assise notamment en tailleur, sans l'aide d'une tierce personne ni d'un appareillage contraignant.

### Technique antérieure :

La position du tailleur correspond à une position assise dans laquelle les jambes sont croisées, les pieds sont sous les genoux opposés, les ischions reposent au sol, la colonne vertébrale et la tête sont droites et alignées. La position du tailleur est une des postures de base du yoga, idéale pour les exercices respiratoires et pour la méditation. Elle favorise l'ouverture des hanches et étire la colonne vertébrale.

Sur le plan orthopédique, la position assise en tailleur place les hanches de la personne en forte abduction, flexion et rotation externe. Elle étire les adducteurs, les extenseurs et les rotateurs internes des hanches. Le sartorius est un muscle permettant de prendre la position assise en tailleur, d'où son nom en ancienne nomenclature : muscle couturier.

Chez les personnes souffrant de dysplasie des hanches ou de déséquilibre musculaire, atteintes de malformations comme le spina bifida, de traumatismes crâniens, de paralysies cérébrales ou de toutes autres pathologies, l'indication de la position ou de l'attitude en tailleur peut être préconisée. Cette position permet entre autre de lutter contre les attitudes vicieuses des membres inférieurs : en ciseaux, en W (Frog-sitting). Elle peut être également indiquée chez la personne ayant des troubles du comportement tel que l'autisme. La position assise en tailleur permet de stabiliser le bassin de la personne handicapée, de redresser le tronc et de la tête, et de créer un effet apaisant. Dans cette position, la personne peut regarder les autres personnes qui l'entourent plutôt que le plafond, ce qui facilite grandement la communication par le regard et le langage.

Depuis de nombreuses années, on élabore des coussins ou des cales en mousse, réalisés sur mesure ou sur moulage, pour accompagner la personne vers une position en tailleur. Mais il n'existe actuellement sur le marché aucune solution globale, ni aucune orthèse visant à prévenir et à corriger les troubles du positionnement global du bassin, du tronc et des membres inférieurs de la personne handicapée grâce à une aide à la position assise en tailleur sans contrainte.

Les publications FR 2 447 169 et US 3,890,004 décrivent des sièges favorisant la position assise en tailleur notamment pour la méditation, sans toutefois assurer un maintien périphérique de la personne. Aucun dispositif connu n'est de fait adapté à une personne handicapée incapable de se tenir en position assise de manière autonome.

On connaît également de la publication US 5 490 717 un siège permettant de s'asseoir en position du lotus. Le siège comporte une zone concave formant le fond et terminée à l'avant par un plot central. La zone concave est définie par une zone arrière inclinée vers le haut et vers l'arrière, qui est terminée à l'avant par une zone avant inclinée vers le haut et vers l'avant, et à l'arrière par un dossier qui est penché vers le haut et vers l'avant. Ces inclinaisons permettent de basculer le bassin de la personne vers l'avant. Le dossier permet de soulager les lombaires de la personne et le plot central d'éviter le glissement de la personne vers l'avant. Le plot central est profilé et comporte deux creux frontaux permettant de faire passer les jambes de la personne de façon surélevée. Toutefois, ce siège ne permet pas de maintenir la personne latéralement.

La publication CA 2 578 639 décrit un fauteuil enclos comportant deux positions, une position ouverte formant un fauteuil et une position fermée formant un fauteuil enclos isolé phoniquement. A cet effet, le fauteuil comporte deux dômes superposés. Le premier dôme forme le couvercle, tandis que le second dôme forme le siège. Toutefois, ce fauteuil ne permet pas de faciliter ni de maintenir la position tailleur pour une personne présentant un handicap.

On connaît également de la publication AU 2011100012 un coussin de médiation en forme de disque comportant deux zones concaves à l'arrière et deux zones situées à l'avant permettant de recevoir les jambes. Ce coussin facilite la position tailleur, mais ne permet pas le maintien de la personne dans cette position, notamment une personne présentant un handicap.

### Exposé de l'invention :

La présente invention vise à apporter une solution au problème évoqué ci-dessus en proposant un siège ergonomique offrant un complément aux orthèses de positionnement, ce siège étant particulièrement stable et sécurisé, de conception simple, déclinable en différentes tailles, polyvalent et adapté aussi bien à des personnes handicapées qu'à des personnes ne présentant pas de troubles du positionnement et/ou du comportement, ce siège étant évolutif par l'ajout d'accessoires. Un autre but de l'invention est un siège qui favorise la stabilisation du tronc et le confort de la personne en position assise en tailleur qui peut ainsi rester dans cette position plus longtemps, améliore le positionnement volontaire de la tête et des membres supérieurs, et favorise ainsi les perceptions sensorielles et les exécutions motrices de la personne.

Dans ce but, l'invention concerne un siège ergonomique du genre indiqué en préambule, caractérisé en ce qu'il comporte un évidement sensiblement central pour accueillir ladite personne en position assise, ledit évidement étant délimité au moins par un fond formant une assise, une paroi arrière formant un dossier situé dans le prolongement dudit fond et au moins deux parois latérales symétriques s'étendant de ladite paroi arrière et reliées audit fond, ladite paroi arrière et lesdites parois latérales sont enveloppantes et agencées pour envelopper respectivement les flancs, le dos et la nuque de la personne et en ce que le fond dudit évidement présente, en vue de dessus, une forme sensiblement trapézoïdale, agencée pour correspondre au trapèze de la position assise en tailleur de ladite personne, dont au moins le petit côté est délimité par ladite paroi arrière et les côtés sont délimités par lesdites parois latérales.

Dans une première forme de réalisation de l'invention, ledit évidement comporte en outre une paroi frontale reliant lesdites parois latérales et reliée audit fond, lesdites parois arrière, latérales et frontale formant une ceinture périphérique autour dudit fond entourant ladite personne, et le grand côté de la forme sensiblement trapézoïdale dudit fond étant délimité par cette paroi frontale. Ainsi, le volume trapézoïdal fermé défini par cet évidement central verrouille la position assise en tailleur de la personne lui permettant de tenir la position sans contrainte, de manière confortable et dans le temps.

Dans une seconde forme de réalisation de l'invention, ledit évidement comporte une ouverture frontale située entre lesdites parois latérales.

Ledit évidement est de préférence incliné vers l'arrière dudit siège. Dans ce cas, le fond dudit évidement, qui peut avoir une surface plane, définit avec un plan horizontale un angle compris entre 0° exclu et 15°, et de préférence égal à 5°. Ledit fond peut définir avec au moins la paroi arrière dudit évidement un angle compris entre 85° et 110° et de préférence égal à 90°.

Au moins le fond dudit évidement peut présenter une surface plane.

Ledit siège ergonomique peut présenter une forme générale sensiblement pyramidale pourvue d'une base dans laquelle s'inscrit le fond dudit évidement.

La base peut comporter une surface d'appui plane permettant la stabilisation dudit siège sur un plan horizontal ou la base peut comporte une surface d'appui de forme complémentaire à celle d'un élément de réception permettant l'inclinaison dudit siège par rapport à l'horizontale.

Dans ce cas, l'une au moins des parois arrière, latérales ou frontale peut présenter une épaisseur décroissante de la base vers le bord supérieur desdites parois de manière à augmenter la stabilité dudit siège.

Dans une forme préférée de l'invention, ledit siège est réalisé au moins en partie en une matière élastique

Il peut également comporter au moins un élément structurel rigide ou semi-rigide, pouvant être au moins une plaque de support s'étendant en-dessous dudit évidement, une coque s'étendant au moins en partie sur lesdites parois arrière, latérales ou frontale délimitant ledit évidement, une rehausse s'étendant en-dessous dudit évidement.

### Description sommaire des dessins :

La présente invention et ses avantages apparaîtront mieux dans la description suivante de plusieurs modes de réalisation donnés à titre d'exemples non limitatifs, en référence aux dessins annexés, dans lesquels :
- la figure 1 est une vue en perspective d'un siège ergonomique selon l'invention,
- la figure 2 est une vue en coupe du siège de la figure 1 selon l'axe II-II de la figure 3,
- la figure 3 est une vue en coupe du siège de la figure 1 selon l'axe II-III de la figure 2,
- la figure 4 est une vue en coupe du siège de la figure 1 selon l'axe IV-IV de la figure 2,
- les figures 5 et 6 montrent une personne assise en tailleur dans le siège de la figure 1 respectivement en vue de côté et en vue de dessus,
- la figure 7 est une vue en perspective du siège de la figure 1 sur une rehausse fixe,
- la figure 8 est une vue en perspective du siège de la figure 1 sur une rehausse mobile,
- la figure 9 est une vue en perspective du siège de la figure 1 équipé de cales amovibles, et
- la figure 10 est une vue en perspective d'une autre variante du siège de la figure 1 avec une ouverture frontale,
- la figure 11 est une vue en coupe d'un siège ergonomique avec un élément de réception selon une variante de l'invention,
- la figure 12 est une vue en perspective du siège de la figure 11,
- la figure 13 est une vue en perspective de l'élément de réception de la figure 12.

### Illustrations de l'invention et meilleure manière de la réaliser :

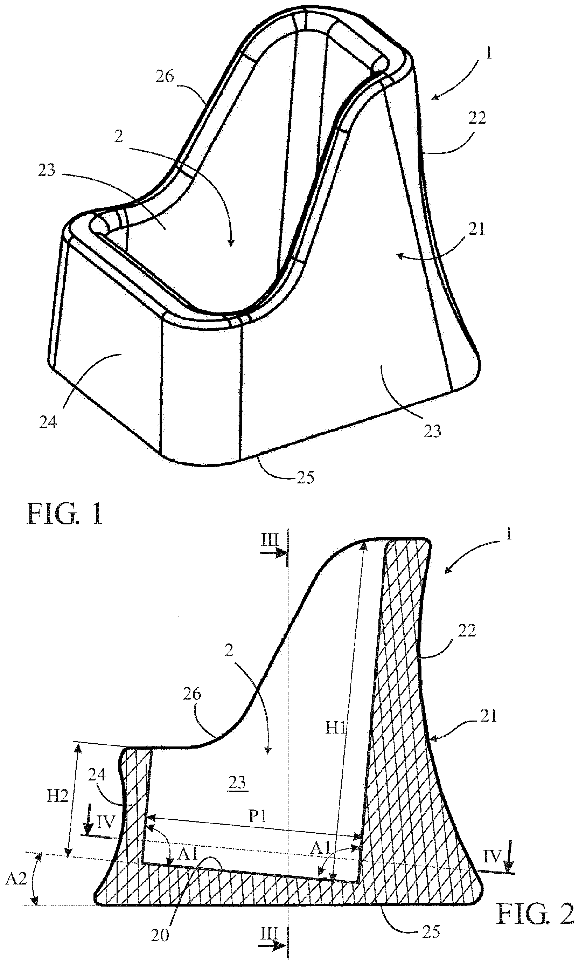

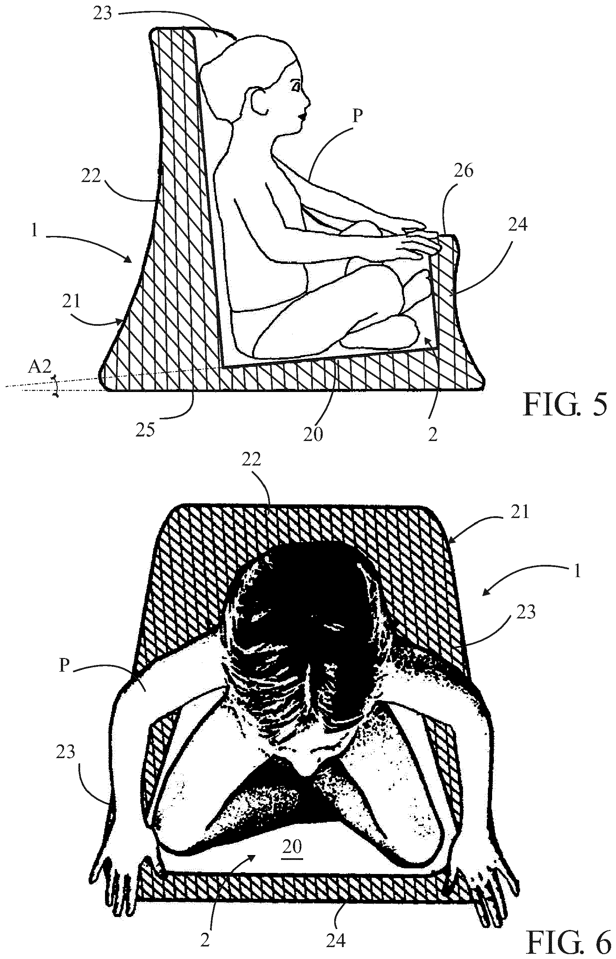

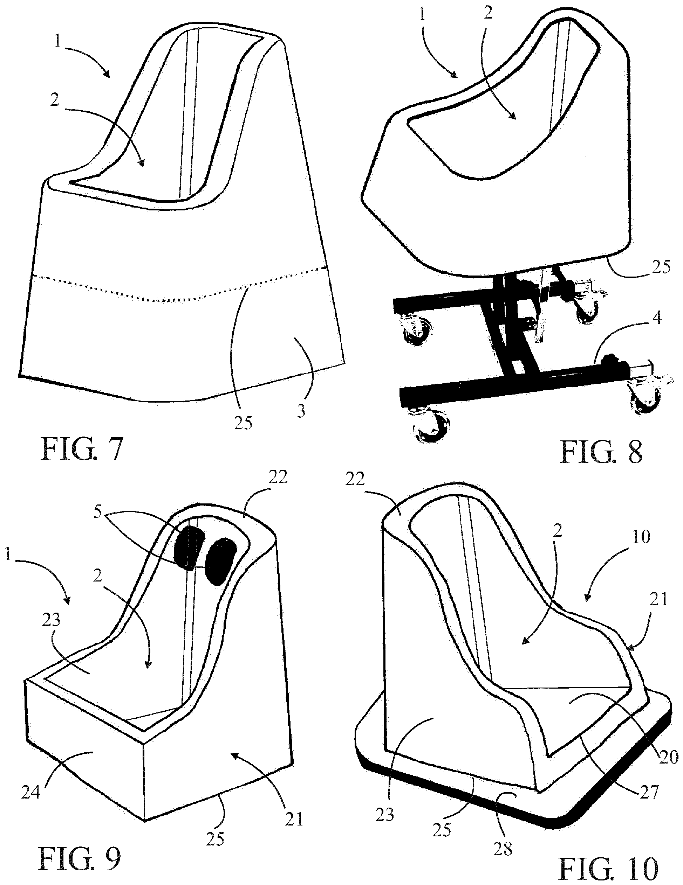

Le siège ergonomique 1 de l'invention et en référence aux figures 1 à 8 comporte un évidement 2 sensiblement central, agencé pour accueillir et envelopper la personne P en position assise en tailleur. Cet évidement 2 est délimité par un fond 20, de préférence plan, formant une assise pour la personne et une ceinture périphérique 21 entourant ledit fond 20 formant une barrière de maintien et de protection autour de la personne. Cette ceinture périphérique 21 comporte une paroi arrière 22 formant un dossier situé dans le prolongement dudit fond 20, deux parois latérales 23 s'étendant de ladite paroi arrière 22 et reliées audit fond 20. Les parois latérales 23 et la paroi arrière 22 sont enveloppantes puisqu'elles enveloppent respectivement les flancs, le dos et la nuque de la personne P, comme le montre la figure 5. En outre, la ceinture périphérique 21 comporte une paroi frontale 24 reliant les deux parois latérales 23 et reliée audit fond 20. Dans ce cas, la ceinture périphérique 21 assure une fonction de maintien de la personne P aussi bien latéral, dorsal, que frontal. Toutefois, la ceinture périphérique 21 peut également être dépourvue de paroi frontale 24, comme cela est illustré à la figure 10. Dans ce cas la ceinture périphérique 21 assure un maintien latéral et dorsal. Ces parois arrière 22, latérales 23 et frontale 24 peuvent être planes ou légèrement courbes, et forment chacune avec ledit fond 20 un angle A1 compris entre 85° et 110° et de préférence égal à 90°. Elles peuvent être reliées entre elles par des angles arrondis ou non et le bord supérieur 26 de la ceinture périphérique 21 peut être courbe, continu et arrondi.

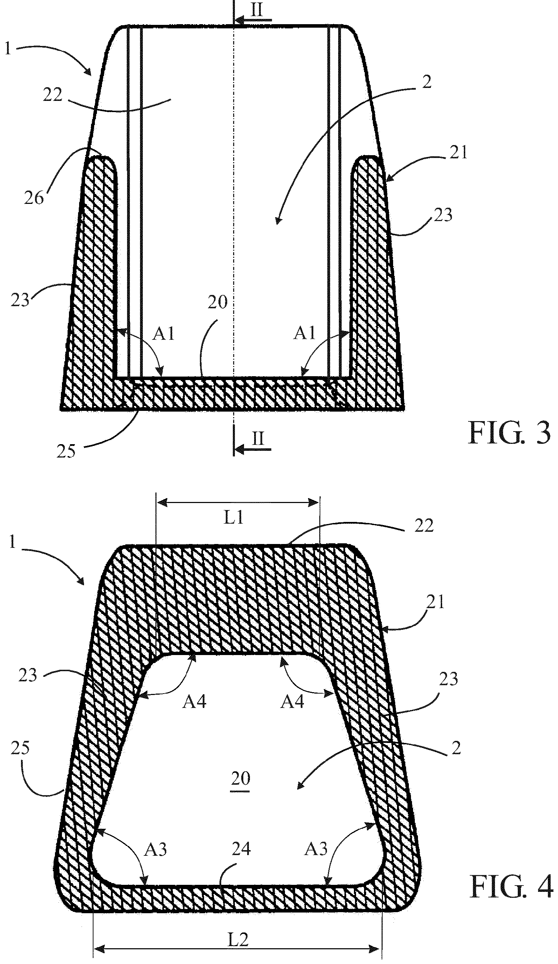

Le siège ergonomique 1 selon l'invention se différencie des sièges connus en ce qu'en vue de dessus, le fond 20 de l'évidement 2 a une forme sensiblement trapézoïdale qui correspond à la forme sensiblement trapézoïdale de la position assise en tailleur de la personne. En effet, la position assise en tailleur représente schématiquement un trapèze dont la petite base est le dos, la grande base les jambes et les pieds en flexion plantaire, et les côtés les cuisses. La forme trapézoïdale du fond 20 comporte une petite base L1 délimitée par la paroi arrière 22, une grande base L2 délimitée par la paroi frontale 24 et deux côtés L3 délimités par les parois latérales. Cette forme trapézoïdale est également définie par ses angles A3 et A4 respectivement entre sa grande base et ses côtés, et entre sa petite base et ses côtés, et par sa profondeur P1 correspondant à la distance entre sa petite base L1 et sa grande base L2 (voir figure 4). Elle est encore définie par ses hauteurs (voir figure 1), notamment la hauteur H1 de sa paroi arrière 22 et la hauteur H2 de sa paroi frontale 24.

Ce siège ergonomique 1 sera proposé en plusieurs tailles pour convenir au plus grand nombre, du bébé à l'adulte. A priori, quatre tailles de siège suffisent à couvrir un large panel de personnes. A titre d'exemple non limitatif, le tableau ci-dessous permet de caractériser ces quatre tailles de siège par les dimensions de l'évidement 2 central accueillant la personne, ces dimensions étant exprimées en centimètres :

| Personne | Taille Siège | Hl-Paroi arrière | H2-Paroi frontale | Ll-Petite base | L2-Grande base | P1 Profondeur |
|---|---|---|---|---|---|---|
| Bébé | 1 | 26 | 9 | 11 | 23 | 18 |
| Enfant | 2 | 40 | 13 | 17 | 35 | 28 |
| Adolescent | 3 | 61 | 20 | 28 | 53 | 42 |
| Adulte | 4 | 93 | 30 | 43 | 80 | 63 |

L'évidement 2 central destiné à accueillir la personne est dans les exemples représentés incliné vers l'arrière du siège 1. Plus précisément, le fond 20 forme avec un plan horizontal ou la base 25 un angle A2 compris entre 0° exclu et 15°, et de préférence égal à 5°. De ce fait, les parois arrière 22 et frontale 24 sont inclinées par rapport à un axe vertical d'un même angle. Cette inclinaison permet à la personne P d'être légèrement basculée vers l'arrière (voir figures 2 et 5). La personne P tient plus facilement et plus longtemps la position assise en tailleur. Plus l'angle A2 est important et plus la position en tailleur est facile à maintenir car les appuis sont mieux répartis sur le fond 20. Il est préférable que cet angle A2 ne dépasse pas les 15° pour éviter le basculement arrière dudit siège 1 par le poids de la personne, sauf à augmenter la stabilité arrière du siège pour ne pas mettre en danger la personne. Dans le cas d'un bébé ou d'un petit enfant, cet angle A2 peut toutefois aller jusqu'à 45°. L'épaisseur de matière entre le fond 20 et la base 25 est également définie en fonction du poids de la personne P pour lui garantir un confort et une souplesse d'assise sans toucher la zone de réception du siège 1.

La paroi arrière 22 permet de verrouiller le positionnement dans le plan antéropostérieur et empêche tout glissement de la personne P vers l'arrière. Sa limite supérieure peut remonter du milieu du dos jusqu'au dessus de la tête. Elle stabilise le dos et limite les mouvements d'hyper-extension du tronc et de la tête. Sa largeur intérieure doit être sensiblement équivalente à la largeur des épaules de la personne P. L'inclinaison de la paroi arrière 22 peut être augmentée pour améliorer le confort de la personne P et l'angle A1 peut être compris entre 85° et 110° et de préférence égal à 90°.

La paroi frontale 24 permet de verrouiller le positionnement dans le plan antéropostérieur et empêche tout glissement de la personne P vers l'avant du siège 1. Elle remonte au dessus des genoux de la personne P assise en tailleur, jusqu'au creux de sa taille par exemple. Si elle est trop basse, la personne P peut s'échapper de la posture, si elle est trop haute, elle peut gêner le champ visuel de la personne P et compliquer son installation par une tierce personne. Son épaisseur est définie en fonction du poids de la personne P. Le bord supérieur 26 de la paroi frontale 24 peut être plat ou légèrement creusé comme sur la figure 7. Il permet d'obtenir un plan de stabilisation horizontal intéressant pour les mains de la personne P ou pour mettre en place une tablette. L'angle A3 qui s'étend entre la paroi frontale 24 et les parois latérales 23 détermine la position assise en tailleur et peut être compris entre 70° et 90° et de préférence égal à 80° (voir figures 4 et 6).

Les parois latérales 23 stabilisent le siège 1 latéralement et empêche la personne P de basculer de coté. L'angle A4 s'étendant entre la paroi arrière 22 et les parois latérales 23 définit la position assise en tailleur et peut être compris entre 90° et 110° et de préférence égal à 100° (voir figures 4 et 6). Le bord supérieur 26 est courbe et rejoint en partie haute la paroi arrière 22 et en partie basse la paroi frontale 24. Si la paroi arrière 22 s'étend au niveau ou au-dessus de la tête de la personne P, les parois latérales 23 s'étendent à l'avant des épaules de la personne P (voir figure 5), enveloppant ainsi la personne latéralement sur toute la hauteur du tronc.

La ceinture périphérique 21 permet ainsi de stabiliser la personne P dans le plan antéropostérieur et latéralement.

Le siège ergonomique 1, 10 selon l'invention peut être réalisé en partie ou en totalité en une matière élastique, telle que notamment une mousse synthétique choisie parmi le polyuréthane, le polyester, le polyéthylène ou similaire. Un des exemples non limitatifs consiste à utiliser une mousse de polyuréthane à haute résilience HR. La densité de la mousse est notamment choisie en fonction des paramètres de la personne tels que son poids, sa taille, sa force musculaire, sa fragilité cutanée. A titre d'exemple non limitatif, on peut citer une mousse d'une densité comprise entre 17kg/m³ et 60kg/m³. Selon le mode d'exécution choisi, ce siège peut être réalisé dans une seule et même matière de densité unique ou dans une même matière de densités différentes ou encore dans des matières différentes avec des densités égales ou différentes, ceci pour renforcer certaines zones par rapport à d'autres en fonction des zones d'appui et de contraintes mécaniques, mais aussi pour réduire le coût matière sans réduire les performances mécaniques du siège, etc.

Ce siège peut être réalisé dans une pièce monobloc qui peut être taillée, découpée ou moulée selon le procédé de fabrication choisi. Il peut aussi être réalisé par un assemblage intime, notamment par collage, de plusieurs pièces qui sont taillées, découpées ou moulées. Il peut encore être modulable et démontable par un assemblage de plusieurs pièces, rendues solidaires entre elles au moyen d'un système de fixation rapide par velcro®, clips réglables ou de tout autre système permettant de rendre démontable ledit siège de manière à faciliter son transport.

Ce siège peut également comporter au moins un élément structurel rigide ou semi-rigide, tel que par exemple une plaque de support 28 (cf. fig. 10) ayant une fonction stabilisatrice, une coque englobant tout ou partie du siège.

La finition de ce siège est bien entendu adaptée à sa destination. Il peut être protégé par une enduction, un revêtement ou une housse. La housse peut être élastique pour ne pas nuire à l'élasticité dudit siège. Elle peut être amovible, lavable, capitonnée, matelassée, réalisée par exemple en cuir, en tissu, en non-tissé, en éponge, en voile ou film de matière synthétique ou composite. Elle peut avoir des propriétés hypoallergiques, respirantes, imperméables. Elle peut être traitée ou non par des additifs anti-tâches, anti-odeurs, ignifugeants, etc. en fonction de la destination dudit siège.

Ainsi, ce siège limite les attitudes vicieuses de la personne et inhibe de manière globale ses mouvements parasites grâce à l'effet élastique de la matière. Le siège va vivre et bouger avec la personne, tout en lui assurant protection et sécurité étant qu'il est conçu pour ne pas basculer même sous l'effet de mouvements brusques. De ce fait, les risques de blessures sont très largement diminués voire supprimés. Ce siège constitue un vrai nouveau positionnement pour la personne handicapée moteur qui peut élargir ses possibilités de changement de position.

Ce siège ergonomique 1 peut présenter, comme dans les exemples illustrés, une forme générale sensiblement pyramidale à quatre côtés. Dans ce cas, il est évasé en direction d'une base 25 formant, en référence aux exemples illustrés dans les figures 1 à 10, une surface d'appui plane et stable sur une zone de réception plane dudit siège, notamment horizontale.

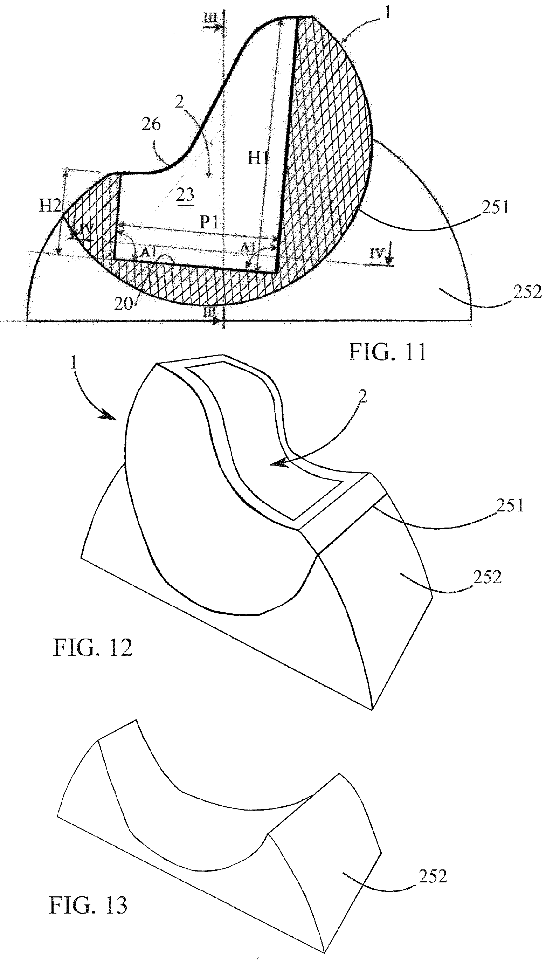

Toutefois, la surface d'appui du siège ergonomique 1 n'est pas obligatoirement plane. En effet, la base 251 peut reposer sur un élément de réception 252 présentant une surface de réception complémentaire avec la surface d'appui de la base 251. Comme l'illustre les figures 11 à 13, la base 251 présente une surface d'appui incurvée convexe et repose sur un élément de réception 252 de surface complémentaire incurvée concave. De cette façon, la base 251 peut être inclinée par rapport à l'horizontale en déplaçant le siège 1 relativement par rapport à l'élément de réception 252 permettant d'offrir une infinité de positions inclinées. L'élément de réception 252 est agencé pour reposer sur une surface plane telle qu'un sol ou une table par exemple. L'élément de réception 252 peut également comporter un élément de renfort (non représenté), tel qu'une planche, disposé à sa base. Cet élément de renfort est agencé pour rigidifier la base et conférer une meilleure stabilité à l'élément de réception 252. Cet agencement est particulièrement intéressant lorsque l'on souhaite que la personne P adopte une position sensiblement inclinée par rapport à l'horizontale, comme c'est par exemple le cas pour des nourrissons, voire des prématurés. Cette configuration n'a bien entendu aucun impact sur la forme de l'évidement 2 décrit précédemment. Dans cet esprit, la base 251 du siège 1 et l'élément de réception 252 peuvent présenter des surfaces d'appui complémentaires différentes de la forme partiellement cylindrique telle que représentée aux figures 11 à 13, comme par exemple une forme partiellement polygonale permettant de poser le siège 1 au choix sur un élément de réception 252 pour pouvoir l'incliner par rapport à l'horizontale, ou sur une surface de réception plane horizontale en dehors de tout élément de réception.

La base 25 du siège 1 peut présenter une forme sensiblement trapézoïdale ou toute autre forme adéquate suffisamment étendue pour englober l'évidement 2 (voir figures 4 et 6) et assurer la stabilité dudit siège 1. Comme représenté sur les figures 2 à 4 en coupe, l'épaisseur de la ceinture périphérique 21 est décroissante en partant de la base 25 vers son bord supérieur 26 contribuant également à augmenter la stabilité du siège du fait d'une plus grande quantité de matière élastique dans la partie basse dudit siège. Comme représenté dans certaines figures, la face externe de la paroi périphérique 21 peut être au moins partiellement incurvée, ceci permettant à la fois de réduire le coût matière et d'augmenter la flexibilité des parois dudit siège. Toutefois, toute autre forme générale de siège peut convenir, comme une forme sensiblement cubique ayant des parois d'épaisseur constante.

La zone de réception dudit siège, de préférence horizontale, peut être un sol, une plateforme, une estrade, une table, etc. selon l'âge de la personne installée dans ledit siège et de l'activité envisagée de ou avec cette personne. Ce siège 1 peut bien entendu être combiné à des accessoires pour multiplier ses possibilités d'applications, tels qu'une rehausse 3 selon la figure 7 sur laquelle est installé ledit siège 1, cette rehausse pouvant être formée d'un bloc de matière tronconique rigide ou élastique, un pied à roulettes 4 selon la figure 8 pourvu d'une plateforme sur laquelle est fixé ledit siège 1 et pouvant être réglable en hauteur. Il peut aussi comporter une plaque de support 28 (voir figure 10) sur laquelle est fixé intimement ledit siège 1, 10 par exemple par collage, cette plaque permettant d'augmenter la stabilité du siège dans son plan d'appui. Tout autre support équivalent peut également convenir.

En complément, un tel siège ergonomique 1 peut comporter selon les besoins et les options choisies :
- des cales de correction amovibles permettant de modifier son inclinaison par rapport à un plan horizontal d'une zone de réception dudit siège,
- des cales souples et amovibles permettant de réduire la dimension de l'évidement 2 de la personne P,
- une tablette permettant de donner un support plus large pour les avant-bras et les mains de la personne P ou pour y déposer des objets d'éveil ou d'éducation motrice,
- une cale ou plusieurs cales 5 pour la nuque et/ou pour la tête, amovibles et fixées par un système de fixation rapide sur la paroi arrière 22 et/ou sur les parois latérales 23 de l'évidement 2, comme illustré à la figure 9,
- une ou plusieurs sangles de fixation de la personne P ou d'un membre de la personne P par rapport audit siège 1,
- une housse de finition de préférence amovible, lavable et imperméable.

Ce siège ergonomique 1 peut bien entendu convenir à tout type de personnes, qui ne sont pas nécessairement handicapées. Il est notamment possible d'élargir les applications de ce siège 1 en proposant un siège 10 dont la ceinture périphérique 21 est ouverte et délimite une ouverture frontale 27 entre les deux parois latérales 23 comme représenté à la figure 10. Dans ce cas, la ceinture périphérique 21 assurant le maintien de la personne, ne comporte pas de paroi frontale, mais une paroi arrière 22 et deux parois latérales 23 enveloppantes. Ce siège 10 ouvert est accessible à toute personne souhaitant adopter différentes positions assises. Notamment, il favorise ou aide la personne à adopter une position assise en tailleur, de manière confortable, stabilisée latéralement, légèrement inclinée vers l'arrière. Ainsi, la position en tailleur est moins fatigante, s'effectue sans tension, et peut être conservé plus longuement.

### Possibilités d'application industrielle :

Comme expliqué plus haut, ce siège ergonomique 1, 10 peut être fabriqué selon différents procédés de découpage, de taille, de moulage ou similaire, en fonction notamment des matières choisies, de la quantité à fabriquer et du prix de revient souhaité.

Ce siège ergonomique sera proposé en plusieurs tailles pour convenir au plus grand nombre, du bébé à l'adulte comme expliqué précédemment.

Il ressort clairement de cette description que l'invention permet d'atteindre les buts fixés. Notamment, le siège ergonomique selon l'invention permet aux personnes souffrant de troubles du positionnement de conserver une posture assise en tailleur de manière indépendante, sans l'aide d'une tierce personne, ni d'un appareillage contraignant. Il offre ainsi une alternative confortable, sans contrainte et conviviale, à une position couchée et à une position assise rigide, contrainte et sanglée. Il améliore le positionnement volontaire de la tête et des membres supérieurs, favorise les perceptions sensorielles et les exécutions motrices grâce à la stabilisation du tronc et des membres inférieurs. Il aide la personne à être actrice de ses capacités. Il inhibe le réflexe d'hyper-extension du tronc et la diffusion des mouvements parasites. Il libère la personne de la nécessité du contrôle volontaire de l'ensemble du corps et lui permet donc de se concentrer sur une tâche à effectuer.

Dans le siège ergonomique selon l'invention, l'équilibre du corps de la personne est amélioré grâce au maintien du tronc et des membres inférieurs. Le corps est moins dispersé, plus rassemblé, sécurisé, contenu, englobé, reposé et décontracté. La ceinture scapulaire est par conséquent plus souple et moins figée. La personne contrôle mieux ses mouvements volontaires des membres supérieurs qui ne sont plus parasités par le reste du corps. La participation aux activités est plus active, la personne est plus concentrée, plus attentive, plus autonome et plus dynamique.

La personne installée à l'intérieur du siège ergonomique selon l'invention est de fait plus disponible aux apprentissages cognitifs. La verbalisation, les coordinations oculo-manuelles et oculo-visuelles sont améliorées du fait du maintien de la tête plus longtemps et plus efficace. La concentration obtenue est nettement meilleure.

La durée et la fréquence des séances d'utilisation doivent toutefois s'adapter à chaque personne, généralement entre 20min et 2h. Elles doivent obligatoirement être placées sous la surveillance d'une tierce personne spécialisée ou non, car elles peuvent nécessiter d'être repositionnées de temps en temps.

La présente invention n'est pas limitée aux exemples de réalisation décrits mais s'étend à toute modification et variante évidentes pour un homme du métier tout en restant dans l'étendue de la protection définie dans les revendications annexées.

## Revendications

1. Siège ergonomique (1, 10) notamment pour une personne souffrant de troubles du positionnement et/ou du comportement, ce siège étant agencé pour soutenir une personne assise notamment en tailleur, sans l'aide d'une tierce personne ni d'un appareillage contraignant, ledit siège (1, 10) comporte un évidement (2) sensiblement central pour accueillir ladite personne (P) en position assise, ledit évidement (2) étant délimité au moins par un fond (20) formant une assise, une paroi arrière (22) formant un dossier situé dans le prolongement dudit fond (20), ledit siège (1, 10) comportant au moins deux parois latérales (23) symétriques s'étendant de ladite paroi arrière (22) et reliées audit fond (20), **caractérisé en ce que** ladite paroi arrière (22) et lesdites parois latérales (23) sont enveloppantes et agencées pour envelopper respectivement les flancs, le dos et la nuque de la personne (P), et **en ce que** le fond (20) dudit évidement (2) présente, en vue de dessus, une forme sensiblement trapézoïdale, agencée pour correspondre au trapèze de la position assise en tailleur de ladite personne, dont au moins le petit côté est délimité par ladite paroi arrière (22) et les côtés sont délimités par lesdites parois latérales (23).

2. Siège ergonomique selon la revendication 1, **caractérisé en ce que** ledit évidement (2) est en outre délimité par une paroi frontale (24) reliant lesdites parois latérales (23) et reliée audit fond (20), lesdites parois arrière, latérales et frontale formant une ceinture périphérique (21) autour dudit fond (20), et **en ce que** le grand côté de la forme sensiblement trapézoïdale dudit fond (20) est en outre délimité par ladite paroi frontale (24).

3. Siège ergonomique selon la revendication 1, **caractérisé en ce que** ledit évidement (2) comporte une ouverture frontale (27) située entre lesdites parois latérales (23).

4. Siège ergonomique selon la revendication 1, **caractérisé en ce que** ledit évidement (2) est incliné vers l'arrière dudit siège et **en ce que** le fond (20) dudit évidement (2) définit avec un plan horizontal un angle (A2) compris entre 0° exclu et 15°, et de préférence égal à 5°.

5. Siège ergonomique selon la revendication 1, **caractérisé en ce que** le fond (20) dudit évidement (2) définit avec au moins la paroi arrière (22) dudit évidement (2) un angle (A1) compris entre 85° et 110° et de préférence égal à 90°.

6. Siège ergonomique selon la revendication 1, **caractérisé en ce qu'**au moins le fond (20) dudit évidement (2) présente une surface plane.

7. Siège ergonomique selon l'une quelconque des revendications précédentes, **caractérisé en ce qu'**il présente une forme générale sensiblement pyramidale pourvue d'une base (25, 251) dans laquelle s'inscrit le fond (20) dudit évidement (2).

8. Siège ergonomique selon la revendication 7, **caractérisé en ce que** ladite base (25) comporte une surface d'appui plane permettant la stabilisation dudit siège sur un plan horizontal.

9. Siège ergonomique selon la revendication 7, **caractérisé en ce que** ladite base (251) comporte une surface d'appui de forme complémentaire à celle d'un élément de réception (252) permettant l'inclinaison dudit siège par rapport à l'horizontale.

10. Siège ergonomique selon la revendication 8, **caractérisé en ce que** l'une au moins des parois arrière (22), latérales (23) ou frontale (24) présente une épaisseur décroissante de la base (25) vers un bord supérieur (26) desdites parois.

11. Siège ergonomique selon l'une quelconque des revendications précédentes, **caractérisé en ce qu'**il est réalisé au moins en partie en une matière élastique.

12. Siège ergonomique selon l'une quelconque des revendications précédentes, **caractérisé en ce qu'**il comporte au moins un élément structurel rigide ou semi-rigide.

13. Siège ergonomique selon la revendication 12, **caractérisé en ce qu'**il comporte au moins une plaque de support (28) s'étendant en-dessous dudit évidement (2).

14. Siège ergonomique selon la revendication 12, **caractérisé en ce qu'**il comporte une coque s'étendant au moins en partie sur lesdites parois arrière (22), latérales (23) ou frontale (24) délimitant ledit évidement (2).

15. Siège ergonomique selon la revendication 12, **caractérisé en ce qu'**il comporte une rehausse (3, 4) s'étendant en-dessous dudit évidement (2).

## Patentansprüche

1. Ergonomischer Sitz (1, 10) insbesondere für eine Person, die an Positionierungs- und/oder Verhaltensstörungen leidet, dieser Sitz ist so eingerichtet, dass er eine sitzende Person, vor allem im Schneidersitz, unterstützt, ohne Unterstützung durch eine dritte Person oder eine einschränkende Vorrichtung, dieser Sitz (1, 10) enthält eine im Wesentlichen zentrale Vertiefung (2) zur Aufnahme dieser Person (P) in sitzender Position, die erwähnte Vertiefung (2) wird dabei zumindest von einem Boden (20) begrenzt, der eine Sitzfläche bildet, einer Rückwand (22), die eine Rückenlehne bildet und sich in der Verlängerung des erwähnte Bodens (20) befindet, der Sitz (1, 10) umfasst dabei mindestens zwei symmetrische Seitenwände (23), die von der erwähnten Rückwand (22) aus verlaufen und mit dem Boden (20) verbunden sind, **dadurch gekennzeichnet, dass** die Rückwand (22) und die Seitenwände (23) umhüllend ausgeführt und so vorgesehen sind, dass sie jeweils die Flanken, den Rücken und den Nacken der Person (P) umschließen, und dass der Boden (20) der erwähnten Vertiefung (2) von oben gesehen im Wesentlichen trapezförmig ist, und so ausgeführt ist, dass er dem Trapez der Position der im Schneidersitz sitzenden Person entspricht, bei dem mindestens die kleine Seite von dieser Rückwand (22) begrenzt ist, während die Seiten von den erwähnten Seitenwänden (23) begrenzt sind.

2. Ergonomischer Sitz nach Anspruch 1, **dadurch gekennzeichnet, dass** die erwähnte Vertiefung (2) außerdem von einer Frontwand (24) begrenzt ist, die die erwähnen Seitenwände (23) verbindet und mit dem Boden (20) verbunden ist, dabei bilden diese Rück-, Seiten und Frontwände einen peripheren Gürtel (21) um den Boden (20) herum und dadurch, dass die lange Seite der im Wesentlichen trapezförmige Form des Bodens (20) außerdem von der erwähnten Frontwand (24) begrenzt wird

3. Ergonomischer Sitz nach Anspruch 1, **dadurch gekennzeichnet, dass** die erwähnte Vertiefung (2) außerdem eine Frontöffnung (27) enthält, die sich zwischen den erwähnten Seitenwänden (23) befindet.

4. Ergonomischer Sitz nach Anspruch 1, **dadurch gekennzeichnet, dass** die erwähnte Vertiefung (2) zum hinteren Bereich dieses Sitzes geneigt ist und dass der Boden (20) dieser Vertiefung (2) mit einer horizontalen Ebene einen Winkel (A2) definiert, der zwischen 0°, nicht eingeschlossen, und 15° betragen kann, am besten ist er gleich 5°.

5. Ergonomischer Sitz nach Anspruch 1, **dadurch gekennzeichnet, dass** der Boden (20) dieser Vertiefung (2) mit der mindestens einen Rückwand (22) dieser Vertiefung (2) einen Winkel (A1) zwischen 85° und 110° bildet. am besten ist er gleich 90°.

6. Ergonomischer Sitz laut Anspruch 1, **dadurch gekennzeichnet, dass** mindestens der Boden (20) dieser Vertiefung (2) eine ebene Oberfläche aufweist.

7. Ergonomischer Sitz nach irgendeinem der vorangehenden Ansprüche, **dadurch gekennzeichnet, dass** er im Wesentlichen eine Pyramidenform hat, versehen mit einer Basis (25, 251), in der der Boden (20) dieser Vertiefung (2) eingelassen ist.

8. Ergonomischer Sitz nach Anspruch 7, **dadurch gekennzeichnet, dass** diese Basis (25) eine ebene Stützfläche aufweist, die eine Stabilisierung dieses Sitzes in horizontaler Ebene erlaubt.

9. Ergonomischer Sitz nach Anspruch 7, **dadurch gekennzeichnet, dass** die erwähnte Basis (251) eine Stützfläche enthält, deren Form komplementär ist zu der eines Aufnahmeelementes (252), das eine Neigung dieses Sitzes bezogen auf die Horizontale ermöglicht.

10. Ergonomischer Sitz nach Anspruch 8, **dadurch gekennzeichnet, dass** mindestens eine der Rück- (22), Seiten- (23) oder Frontwände (24) eine von der Basis (25) zu einem oberen Rand (26) dieser Wände abnehmende Dicke aufweist.

11. Ergonomischer Sitz nach irgendeinem der vorangehenden Ansprüche, **dadurch gekennzeichnet, dass** er mindestens zum Teil aus einem elastischen Material ausgeführt ist.

12. Ergonomischer Sitz nach irgendeinem der vorangehenden Ansprüche, **dadurch gekennzeichnet, dass** er mindestens ein starres oder halb-starres Strukturelement enthält.

13. Ergonomischer Sitz nach Anspruch 12, **dadurch gekennzeichnet, dass** er mindestens eine Stützplatte (28) enthält, die unterhalb dieser Vertiefung (2) verläuft.

14. Ergonomischer Sitz nach Anspruch 12, **dadurch gekennzeichnet, dass** er eine Schale enthält, die mindestens zum Teil über diese Rück- (22), Seiten- (23) oder Frontwände (24) verläuft und diese Vertiefung (2) begrenzt.

15. Ergonomischer Sitz nach Anspruch 12, **dadurch gekennzeichnet, dass** er eine Erhöhung enthält (3, 4), die unterhalb dieser Vertiefung (2) verläuft.

## Claims

1. Ergonomic seat (1, 10), in particular for a person suffering from positioning and/or behavioral disorders, this seat being designed to support a seated person, in particular cross-legged, without the aid of a third person or a constraining device, the said seat (1, 10) comprising a substantially central recess (2) for receiving the said person (P) in a seated position, the said recess (2) being delimited by at least a bottom (20) forming a seat, a rear wall (22) forming a backrest forming an extension of the said bottom (20), the said seat (1, 10) comprising at least two symmetrical side walls (23) extending from the said rear wall (22) and connected to the said bottom (20), **characterized in that** the said rear wall (22) and the said side walls (23) are wrap-around and arranged to wrap around respectively the sides, the back and the nape of the neck of the person (P), and **in that** the bottom (20) of the said recess (2) has, in top view, a substantially trapezoidal shape, arranged to correspond to the trapezium of a person sitting cross-legged, the short side of which is bounded by the said rear wall (22) and the sides of which are bounded by the said side walls (23).

2. Ergonomic seat according to claim 1, **characterized in that** the said recess (2) is further delimited by a front wall (24) connecting together the said side walls (23) and connected to the said bottom (20), the said rear, side and front walls forming a peripheral belt (21) around the said bottom (20), and **in that** the long side of the substantially trapezoidal shape of the said bottom (20) is further delimited by the said front wall (24).

3. Ergonomic seat according to claim 1, **characterized in that** the said recess (2) comprises a front opening (27) located between said side walls (23).

4. Ergonomic seat according to claim 1, **characterized in that** the said recess (2) is inclined towards the rear of the said seat and **in that** the bottom (20) of the said recess (2) defines with a horizontal plane an angle (A2) of between 0° excluded and 15°, and preferably of 5°.

5. Ergonomic seat according to claim 1, **characterized in that** the bottom (20) of the said recess (2) defines with at least the rear wall (22) of the said recess (2) an angle (Al) between 85° and 110° and preferably of 90°.

6. Ergonomic seat according to claim 1, **characterized in that** at least the bottom (20) of the said recess (2) has a flat surface.

7. Ergonomic seat according to any one of the preceding claims, **characterized in that** it has a substantially pyramidal general shape provided with a base (25, 251) in which the bottom (20) of the said recess (2) is contained.

8. Ergonomic seat according to claim 7, **characterized in that** the said base (25) comprises a flat support surface allowing the stabilization of the said seat on a horizontal plane.

9. Ergonomic seat according to claim 7, **characterized in that** the said base (251) comprises a bearing surface having a shape complementary to that of a receiving element (252) allowing the inclination of the said seat from horizontal.

10. Ergonomic seat according to claim 8, **characterized in that** at least one of the rear (22), side (23) or front (24) walls has a thickness decreasing from the base (25) towards an upper edge (26) of the said walls.

11. Ergonomic seat according to any of the above claims, **characterized in that** it is made at least partly of an elastic material.

12. Ergonomic seat according to any of the preceding claims, **characterized in that** it comprises at least one rigid or semi-rigid structural element.

13. Ergonomic seat according to claim 12, **characterized in that** it comprises at least one support plate (28) extending below the said recess (2).

14. Ergonomic seat according to claim 12, **characterized in that** it comprises a shell extending at least partly on the said rear (22), side (23) or front (24) walls delimiting the said recess (2).

15. Ergonomic seat according to claim 12, **characterized in that** it comprises a raiser (3, 4) extending below the said recess (2).
